# EUROPÄISCHE PATENTSCHRIFT

(11) **EP 0 945 103 B1**
(45) Veröffentlichungstag und Bekanntmachung des Hinweises auf die Patenterteilung: **12.06.2002**
(21) Anmeldenummer: 99104920.6
(22) Anmeldetag: 11.03.1999
(51) Int. Cl.: A61B 6/00, A61B 5/05, A61B 6/04

(54) **Vorrichtung zur Untersuchung von lebendem Gewebe**
Apparatus for examining living tissue
Dispositif pour examen de tissu vivant

(30) Priorität: 24.03.1998 DE 19812974; 10.02.1999 DE 19905532
(43) Veröffentlichungstag der Anmeldung: 29.09.1999
(73) Patentinhaber: SIEMENS AKTIENGESELLSCHAFT, 80333 München (DE)
(72) Erfinder: Mertelmeier, Thomas Dr., 91058 Erlangen (DE); Scholz, Bernhard Dr., 91336 Heroldsbach (DE)

(56) Entgegenhaltungen:
- DE-A- 3 017 168
- US-A- 4 291 708
- US-A- 5 664 573
- PIPERNO G ET AL: "BREAST CANCER SCREENING BY IMPEDANCE MEASUREMENTS" FRONTIERS OF MEDICAL AND BIOLOGICAL ENGINEERING, Bd. 2, Nr. 2, 1. Januar 1990, Seiten 111-117, XP000676951
- ROWSON J ET AL: "SECOND-GENERATION COMPILERS OPTIMIZE SEMICUSTOM CIRCUITS" ELECTRONIC DESIGN, Bd. 35, Nr. 4, 19. Februar 1987, Seiten 92-96, XP000003705

## Beschreibung

Die Erfindung betrifft eine Vorrichtung zur Untersuchung von lebendem Gewebe.

Solche Vorrichtungen werden beispielsweise verwendet, um die weibliche Brust auf Tumore zu untersuchen.

Bei einer aus der US 4 291 708 bekannten Vorrichtung ist eine Elektrodenanordnung vorgesehen, die mit dem zu untersuchenden Gewebe in elektrischer Wechselwirkung steht. Außerdem sind Mittel zur Beaufschlagung der Elektrodenanordnung mit Stromsignalen und Mittel zum Messen von durch die Stromsignale in das zu untersuchende Gewebe induzierten Potentialen vorgesehen, die ebenfalls mit der Elektrodenanordnung zusammenwirken. Aus den so ermittelten Meßwerten werden für unterschiedliche Bereiche der Brust die dielektrischen Konstanten ermittelt, um aus den Änderungen der die elektrischen Konstante auf die An- oder Abwesenheit eines Tumors schließen zu können.

Vorteilhaft an dieser Vorrichtung ist, daß sie ohne ionisierende Strahlung und nicht invasiv arbeitet. Erfahrungen mit derartigen Vorrichtungen haben jedoch gezeigt, daß im Vergleich zu der klassischen Röntgen-Mammographie Sensitivität und Spezifität zu wünschen übrig lassen.

Bekannte Mammographie-Röntgengeräte weisen eine Röntgenstrahlenquelle, eine der Röntgenstrahlenquelle gegenüberliegend angeordnete Strahlenempfangsanordnung für von der Röntgenstrahlungsquelle ausgehende Röntgenstrahlung und eine zwischen der Röntgenstrahlenquelle und der Strahlenempfangseinrichtung angeordnete Kompressionseinrichtung für das zu untersuchende Gewebe auf.

Aus der US 5 664 573 ist ein Gerät bekannt, das die Durchführung sowohl von Ultraschall- als auch von Röntgenuntersuchungen ermöglicht, wozu in die Kompressionseinrichtung eines Mamographie-Röntgengeräts ein röntgentransparenter Ultraschallwandler integriert ist.

Der Erfindung liegt die Aufgabe zugrunde, eine Vorrichtung der eingangs genannten Art so auszubilden, daß eine für das zu untersuchende Lebewesen möglichst schonende und zugleich zuverlässige Untersuchung möglich ist.

Nach der. Erfindung wird diese Aufgabe durch die Vorrichtung gemäß dem Patentanspruch 1 gelöst.

Mittels der Erfindung ist es also möglich, mit einem einzigen Gerät lebendes Gewebe wahlweise nur durch Röntgenstrahlung oder nur durch elektrische Impedanzmessung oder durch Röntgenstrahlung und elektrische Impedanzmessung zu untersuchen, wobei in letzterem Falle die Untersuchungen durch Röntgenstrahlung und elektrische Impedanzmessung wahlweise sequentiell oder wenigstens im wesentlichen gleichzeitig erfolgen können.

Damit ist es möglich, eine dem jeweiligen Untersuchungsfall hinsichtlich Belastung des zu untersuchenden Lebewesens und Zuverlässigkeit gut angepaßte Untersuchungsprozedur durchzuführen. In diesem Zusammenhang ist von besonderem Vorteil, daß in Fällen, in denen sowohl durch Röntgenstrahlung als auch durch elektrische Impedanzmessung untersucht wird, daß das zu untersuchende Gewebe seine Position in der Kompressionseinrichtung beibehalten kann, so daß beide Untersuchungsergebnisse optimal vergleichbar sind. Dies ist offensichtlich dann nicht der Fall, wenn mit zwei getrennten Geräten gearbeitet werden muß, da es praktisch unmöglich ist, das zu untersuchende Gewebe in beiden Geräten in der gleichen Weise zu komprimieren.

Die Impedanzmessung kann durch Beaufschlagung mit Stromsignalen und Messung der induzierten Potentiale oder alternativ durch Beaufschlagung mit Spannungssignalen und Messung der induzierten Ströme erfolgen.

Um zu verhindern, daß die zur Impedanzmessung erforderlichen Einzelelektroden die Röntgenbilder nachteilig beeinflussen, ist gemäß einer Ausführungsform der Erfindung vorgesehen, daß die Einzelelektroden aus einem Röntgenstrahlung wenig schwächenden Material gebildet sind. Um nachteilige Einflüsse der Elektrodenanordnung auf die Röntgenbilder gänzlich zu vermeiden, sieht eine besonders bevorzugte Ausführungsform der Erfindung vor, daß die Elektrodenanordnung einen elektrisch nicht aktiven Bereich aufweist, der derart geformt ist, daß er bei Abbildung mittels der von der Röntgenstrahlungsquelle ausgehenden Röntgenstrahlung auf der Strahlenempfangseinrichtung ein dem Bild der Einzelelektroden inverses Bild ergibt, das den gleichen Schwärzungsgrad wie das Bild der Einzelelektroden aufweist. In diesem Falle findet also zwar eine gewisse Schwächung der Röntgenstrahlung durch die Elektrodenanordnung statt, jedoch ist das Vorhandensein der Elektrodenanordnung in den Röntgenbildern praktisch nicht erkennbar.

Bevorzugte Ausführungsformen der Erfindung sehen vor, daß eine Recheneinrichtung vorgesehen ist, die auf Grundlage von den mittels der Mittel zum Messen von induzierten Potentialen gemessenen Potentialen bzw. den mittels der Mittel zum Messen von induzierten Strömen gemessenen Strömen entsprechenden digitalen Daten eine vorzugsweise dreidimensionale Verteilung von Impedanzwerten errechnet, wobei die Recheneinrichtung die Verteilung von Impedanzwerten, vorzugsweise durch ein Rekonstruktionsverfahren, errechnet. Geeignete Rekonstruktionsverfahren sind in "A review of image reconstruction techniques for electrical impedance tomography" D.C. Barber, Med. Phys., Vol. 16, No. 2, Mar/Apr. 1989, Am. Assoc. Phys. Med., S. 162-169, beschrieben.

Eine weitere besonders bevorzugte Ausführungsform der Erfindung sieht vor, daß die Recheneinrichtung unter Berücksichtigung der der Erzeugung des Röntgenbildes und der Ermittlung der dreidimensionalen Verteilung der Impedanzwerte zugrundeliegenden Koordinatensysteme eine Fusion zumindest eines Teils der dreidimensionalen Verteilung der Impedanzwerte mit dem Röntgenbild entsprechenden digitalen Daten zu einem Fusionsbild bewirkt. Dabei erfolgt die Erzeugung des Fusionsbildes in vorteilhafter Weise in der Weise, daß die Recheneinrichtung die dreidimensionaler Verteilung der Impedanzwerte in das Röntgenbild projiziert, wobei auch die Möglichkeit besteht, nur eine oder mehrere Schichten der dreidimensionalen Verteilung der Impedanzwerte in das Röntgenbild zu projizieren.

Weiter besteht gemäß einer Variante der Erfindung die Möglichkeit, daß die Recheneinrichtung das Fusionsbild durch Einblendung des Röntgenbildes in eine der Schichten der dreidimensionalen Verteilung der Impedanzwerte erzeugt.

Vorzugsweise handelt es sich bei der Strahlenempfangseinrichtung um eine Strahlenempfangseinrichtung, die dem Röntgenbild entsprechende digitale Daten liefert, die einer Recheneinrichtung zugeführt sind. Es kann aber auch vorgesehen sein, daß die Strahlenempfangseinrichtung dem Röntgenbild entsprechende analoge Signale liefert und daß Mittel zur Konvertierung der analogen Signale in dem Röntgenbild entsprechende digitale Daten vorgesehen sind, die einer Recheneinrichtung zugeführt sind. Schließlich kann die Strahlenempfangseinrichtung durch einen Film für Röntgenbilder gebildet sein, wobei eine Digitalisierungseinrichtung vorgesehen ist, die den belichteten Film abtastet und dem Röntgenbild entsprechende digitale Daten liefert, die einer Recheneinrichtung zugeführt sind.

Ein Ausführungsbeispiel der Erfindung ist in den beigefügten schematischen Zeichnungen dargestellt. Es zeigen:
- Fig. 1: eine erfindungsgemäße Vorrichtung in teilweise blockschaltbildartiger Darstellung,
- Fig. 2: eine Ansicht einer ersten Schicht der Elektrodenanordnung des Gerätes gemäß Fig. 1,
- Fig. 3: eine Ansicht einer zweiten Schicht der Elektrodenanordnung des Gerätes gemäß Fig. 1,
- Fig. 4: einen Teil der Kompressionseinrichtung des Gerätes gemäß Fig. 1 im Querschnitt, und
- Fig. 5: ein die Bildverarbeitung im Falle des Gerätes gemäß Fig. 1 veranschaulichendes Diagramm.

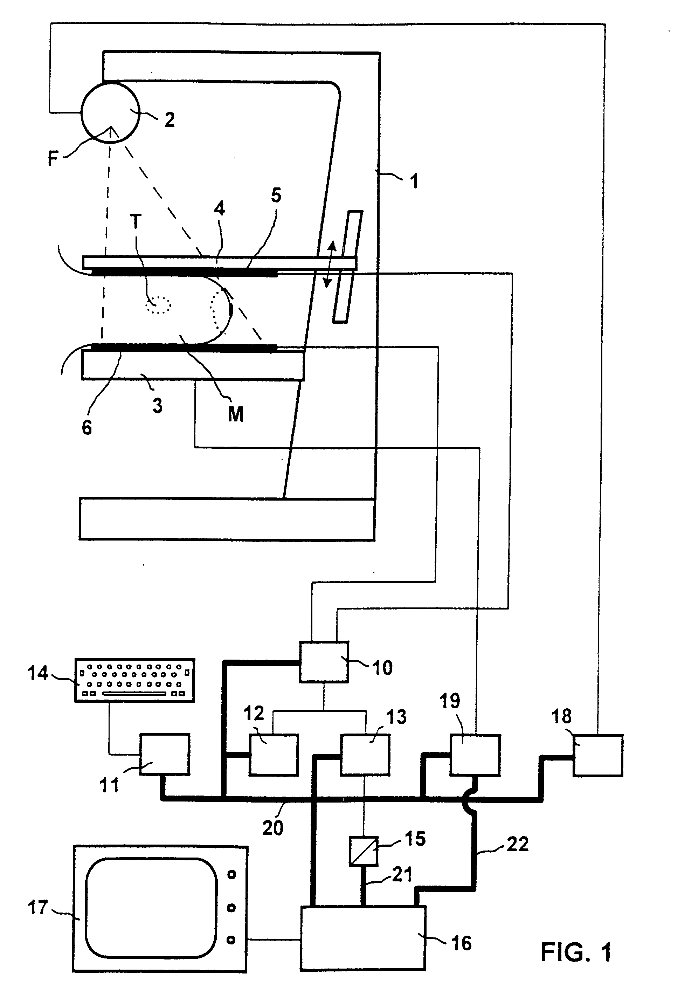

Die in Fig. 1 dargestellte Vorrichtung dient dazu, Tumore in lebendem Gewebe zu detektieren und weist einen Ständer 1 auf, an dem einander gegenüberliegend ein Röntgenstrahler 2 und als Strahlenempfangsanordnung ein Strahlenempfänger 3 derart angebracht sind, daß das von dem Fokus F des Röntgenstrahlers 2 ausgehende, strichliert angedeutete Röntgenstrahlenbündel auf dem Strahlenempfänger 3 auftrifft.

Bei dem Strahlenempfänger 3 handelt es sich um einen solchen Typ, der dem empfangenen Röntgenbild entsprechende digitale Signale abgibt, also z.B. einen Flachbildwandler auf der Basis von amorphem Silizium (a:Si).

Zwischen dem Röntgenstrahler 2 und dem Strahlenempfänger 3 ist eine aus einem für Röntgenstrahlung gut durchlässigen Material, z.B. gebildete Kompressionsplatte 4 an dem Ständer 1 derart verstellbar angeordnet, daß zu untersuchendes Gewebe, beispielsweise wie in Fig. 1 dargestellt eine einen Tumor enthaltende weibliche Mamma M, zwischen dem Strahlenempfänger 3 und der Kompressionsplatte 4 komprimiert werden kann. Die Kompressionsplatte 4 und der Strahlenempfänger 3 bilden also eine Kompressionseinrichtung.

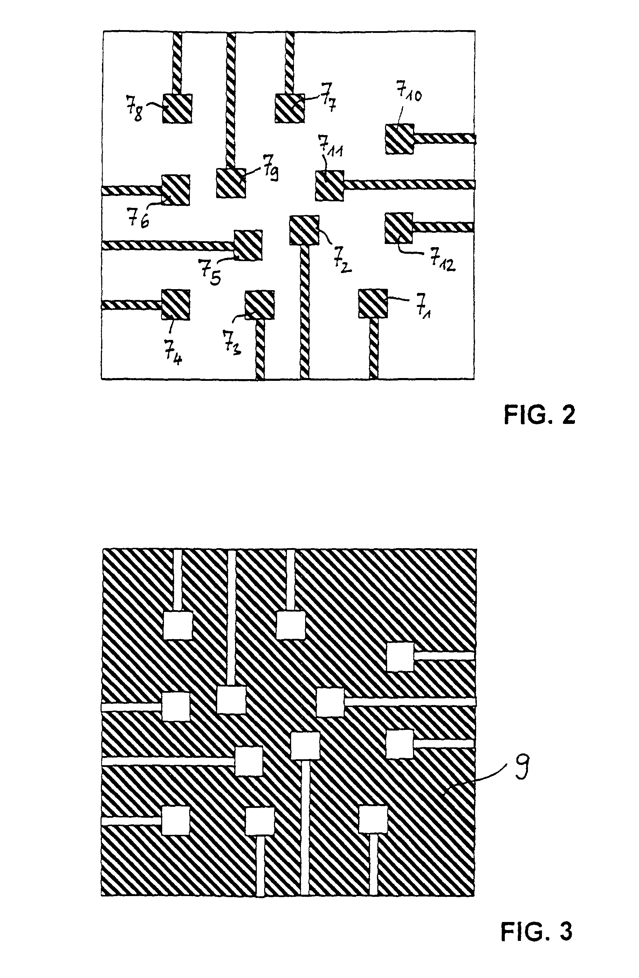

An den einander gegenüberliegenden Flächen des Strahlenempfängers 3 und der Kompressionsplatte 4 sind in den Fig. 2 bis 4 näher veranschaulichte Elektrodenanordnungen 5, 6 vorgesehen, die mit dem zwischen dem Strahlenempfänger 3 und der Kompressionsplatte 4 komprimierten Gewebe in elektrischer Wechselwirkung stehen. Diese elektrische Wechselwirkung kann beispielsweise durch ein Gel gewährleistet werden.

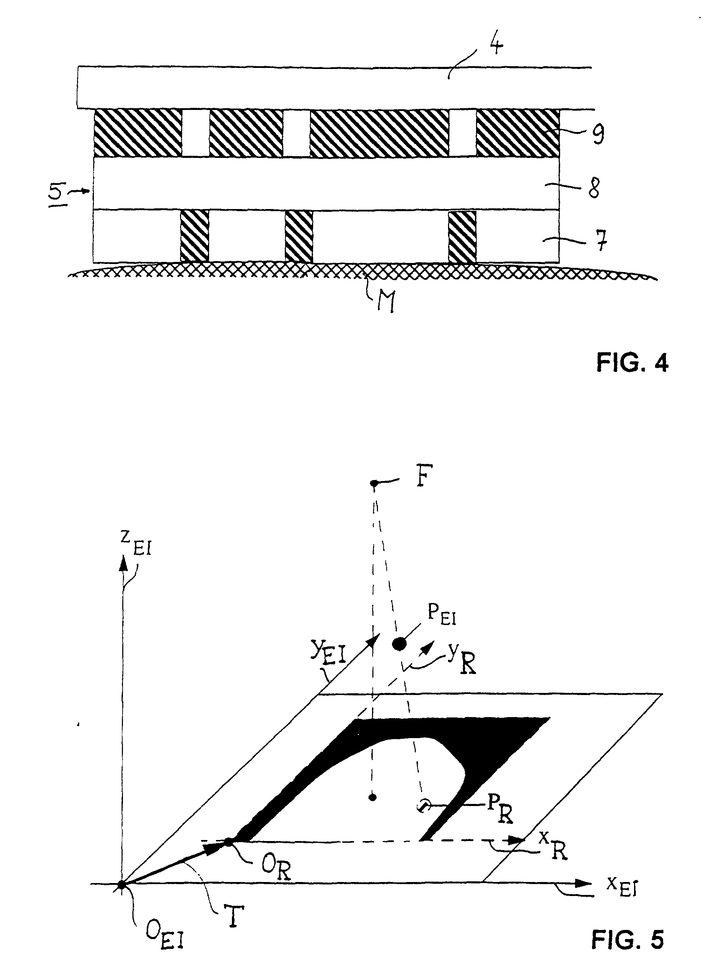

Wie aus der Fig. 4 am Beispiel der Elektrodenanordnung 5, die Elektrodenanordnung 6 ist entsprechend aufgebaut, ersichtlich ist, ist die Elektrodenanordnung 5 aus drei Schichten gebildet und an der Kompressionsplatte 4 angebracht. Bei der in Fig. 2 untersten Schicht 7 handelt es sich um elektrisch aktive Einzelelektroden, die in noch näher zu beschreibender Weise zur elektrischen Impedanzmessung verwendet werden. Ein Beispiel für eine solche Anordnung der Einzelelektroden 7₁ bis 7₁₂ ist in Fig. 3 veranschaulicht.

Anschließend ist eine Isolierschicht 8 vorgesehen, die die Einzelelektroden 7₁ bis 7₁₂ von einem die dritte Schicht der Elektrodenanordnung 5 bildenden, elektrisch nicht aktiven Bereich 9 trennt, der in der aus der Fig. 3 ersichtlichen Weise derart geformt ist, daß er zu der Anordnung der Einzelelektroden 7₁ bis 7₁₂ invers ist.

Die Einzelelektroden 7₁ bis 7₁₂ sind ebenso wie der elektrisch nicht aktive Bereich 9 der Elektrodenanordnung 5 beispielsweise aus folienartigem Leitermaterial aus Kohlenstoff gebildet. Auch bei der Isolierschicht 8 handelt es sich um ein folienartiges Material, beispielsweise Zelluphan. Man kann sich die Elektrodenanordnung 5 also als folienartige gedruckte Schaltung vorstellen, die mit der Kompressionsplatte 4, beispielsweise durch Kleben, verbunden ist. Geeignete Elektroden werden z.B. von den Firmen Joiko, Deutschland, und Medicotest, Dänemark, angeboten.

Da die Einzelelektroden 7₁ bis 7₁₂ und der elektrisch nicht aktive Bereich der Elektrodenanordnung 5 nicht nur aus dem gleichen Material gebildet sind, sondern auch die gleiche Dicke aufweisen, ergibt sich bei Abbildung des elektrisch nicht aktiven Bereichs mittels der von dem Röntgenstrahler 2 ausgehenden Röntgenstrahlung ein dem Bild der Einzelelektroden 7₁ bis 7₁₂ inverses Bild, das den gleichen Schwärzungsgrad wie das Bild der Einzelelektroden 7₁ bis 7₁₂ aufweist. Da die Elektrodenanordnung 5 aus dünnen Folien besteht, insgesamt also sehr dünn ist, wirkt sich auch die perspektivische Röntgenprojektion nicht auf die Nichtsichtbarkeit aus.

Die Elektrodenanordnung 5, und das gleiche gilt für die entsprechend aufgebaute Elektrodenanordnung 6, ist also in einem mittels des Röntgenstrahlers 2 und des Strahlenempfängers 3 erzeugten Röntgenbild nicht sichtbar.

Wie die Fig. 1 schematisch zeigt, sind die Elektrodenanordnungen 5 und 6, und zwar deren Einzelelektroden, über eine Umschalteinheit 10, die von einer Steuereinheit 11 betätigt werden kann, wahlweise mit Mitteln zur Beaufschlagung wenigstens eines Teils der Einzelelektroden mit elektrischen Signalen, nämlich einem Impulsgenerator 12, oder Mitteln zum Messen von durch diese elektrische Signale in das zu untersuchende Gewebe induzierten elektrischen Signalen, nämlich einer Detektorschaltung 13, verbindbar. In Fig. 1 ist diese Anordnung aus Gründen der Übersichtlichkeit einkanalig dargestellt, in der Praxis muß die Anordnung jedoch mehrkanalig ausgeführt sein, wobei die Anzahl der Kanäle im Höchstfall der Anzahl der Einzelelektroden entsprechen kann.

Mittels einer an die Steuereinheit 11 angeschlossenen Tastatur 14 kann eine Betriebsart eingestellt werden, in der der Impulsgenerator 12 Stromsignale abgibt und die Detektorschaltung 13 die infolge der Stromsignale induzierten Potentiale mißt. Alternativ kann mittels der Tastatur 11 eine andere Betriebsart gewählt werden,in der der Impulsgenerator 12 Spannungssignale abgibt und die Detektorschaltung 13 die infolge der Spannungssignale induzierten Ströme mißt.

Die Elektrodenanordnungen 5, 6, die Steuereinheit 11, die Umschalteinheit 11, der Impulsgenerator 12 und die Detektoreinheit 13 wirken über einen Steuerbus 20 zur Durchführung einer Untersuchung derart zusammen, daß aufeinanderfolgend mittels der Umschalteinheit unterschiedliche Kombinationen von Einzelelektroden mit dem Impulsgenerator 12 verbunden und von diesem mit impulsartigen elektrischen Signalen beaufschlagt werden, während andere Einzelelektroden über die Umschalteinheit 10 mit der Detektoreinheit 13 verbunden sind, so daß am Ausgang der Detektoreinheit 13 Signale zur Verfügung stehen, die den in das zu untersuchende Gewebe infolge der impulsartigen elektrischen Signale induzierten elektrischen Signalen entsprechen. Die impulsartigen elektrischen Signale können in an sich bekannter Weise mono- oder multifrequent sein.

Das Muster, nach dem aufeinanderfolgend Einzelelektroden mit impulsartigen elektrischen Signalen beaufschlagt werden, kann mittels der Tastatur 14 eingegeben bzw. aus einer Anzahl von in der Steuereinheit 11 gespeicherten Mustern ausgewählt werden.

Die Ausgangssignale der Detektoreinheit 13 gelangen über einen Analog/Digital-Wandler 15 und eine Datenleitung 21 zu einer elektronischen Recheneinrichtung, nämlich dem Bildrechner 16, der aus den den Ausgangssignalen der Detektoreinheit 13 entsprechenden digitalen Daten nach dem Algorithmus eines an sich bekannten Rekonstruktionsverfahrens eine dreidimensionale Verteilung von Impedanzwerten, z.B. der dielektrischen Konstante, der Leitfähigkeit u. dgl., berechnet und speichert.

Diese dreidimensionale Verteilung der Impedanzwerte oder vorzugsweise ebene Schichten der dreidimensionalen Verteilung der Impedanzwerte können auf einer an den Bildrechner 16 angeschlossenen Anzeigeeinrichtung 17 dargestellt werden, nachdem die Impedanzwerte von dem Bildrechner 16 in entsprechende Grau- oder Farbwerte umgesetzt wurden.

Der Röntgenstrahler 2, ein diesem zugeordneter Hochspannungs-Generator 18, der Strahlenempfänger 3 und die Steuereinheit 11 wirken über einen Steuerbus 20 derart zusammen, daß der Hochspannungs-Generator 18 den Röntgenstrahler 2 mit Werten für Betriebsparameter Röhrenspannung, Röhrenstrom und mAs-Produkt aktiviert, die über die Tastatur 14 einstellbar sind oder einem von mehreren mittels der Tastatur 14 wählbaren in der Steuereinheit 11 gespeicherten Parametersätzen für die genannten Betriebsparameter entsprechen. Die dann am Ausgang des Strahlenempfängers 3 zur Verfügung stehenden, dem aufgenommenen Röntgenbild entsprechenden digitalen Daten gelangen über eine Datenaufbereitungsschaltung 19 und eine Datenleitung 22 zu dem Bildrechner 16, der die dem Röntgenbild entsprechenden digitalen Daten speichert und ggf. nach Durchführung einer Bildnachverarbeitung auf der Anzeigeeinrichtung 17 als Grau- oder Farbwerte darstellen kann.

Mittels der Tastatur 14 sind unterschiedliche Betriebsarten der Vorrichtung wählbar, nämlich eine erste Betriebsart, in der ausschließlich eine elektrische Impedanzmessung stattfindet, eine zweite Betriebsart, in der ausschließlich eine Röntgenuntersuchung erfolgt, und eine dritte Betriebsart, in der eine Röntgenuntersuchung und eine elektrische Impedanzmessung gleichzeitig durchgeführt werden.

Damit ist es möglich, dem jeweiligen Untersuchungsfall gut angepaßte Untersuchungsabläufe zu erstellen und durchzuführen.

Da in dem Bildrechner 16 sowohl die dreidimensionale Verteilung der Impedanzwerte als auch die dem Röntgenbild entsprechenden digitalen Daten gespeichert sind, ist der Bildrechner 16 in der Lage, unter Berücksichtigung der der Erzeugung des Röntgenbildes und der Ermittlung der dreidimensionalen Verteilung der Impedanzwerte zugrundeliegenden Koordinatensysteme, eine Fusion zumindest eines Teils der dreidimensionalen Verteilung der Impedanzwerte mit dem Röntgenbild entsprechenden digitalen Daten zu einem Fusionsbild zu bewirken.

Wie aus der Fig. 5 ersichtlich ist, beziehen sich die einzelnen Werte der dreidimensionalen Verteilung der Impedanzwerte auf ein räumliches rechtwinkliges Koordinatensystem mit den Achsen x_{EI}, y_{EI} und z_{EI} und dem Ursprung O_{EI}. Bei den dem Röntgenbild entsprechenden digitalen Daten handelt es sich um eine ebene Verteilung von Pixeln, in einem rechtwinkligen ebenen Koordinatensystem mit den Achsen y_{R} und x_{R}, und dem Ursprung O_{R} die parallel zu den Achsen y_{EI} und x_{EI} verlaufen und in der gleichen Ebene wie diese liegen. Die beiden Koordinatensysteme hängen also über den Translationsvektor T zusammen.

Um ein Fusionsbild zu erzeugen, projiziert der Bildrechner 16 Werte der dreidimensionalen Verteilung der Impedanzwerte nach den Gesetzen der Zentralprojektion, ausgehend von einem Projektionszentrum Z, dessen Position relativ zu der x_{R}-y_{R}-Ebene der Position des Fokus F des Röntgenstrahlers relativ zu dem Strahlenempfänger 3 entspricht, in das Röntgenbild, so wie dies in der Fig. 5 für einen Wert P_{EI} der dreidimensionalen Verteilung der Impedanzwerte veranschaulicht ist, der dann in dem Röntgenbild an der Stelle P_{R} abgebildet wird.

Es wird also ein Effekt erzielt, der sozusagen eine Abbildung der dreidimensionalen Verteilung der Impedanzwerte mittels des Röntgenstrahlers 2 auf den Strahlenempfänger 3 entspricht.

Dabei besteht in Abhängigkeit von entsprechenden Eingaben mittels der Tastatur 14 die Möglichkeit, die dreidimensionale Verteilung der Impedanzwerte in ihrer Gesamtheit oder nur dreidimensionale Untermengen der dreidimensionalen Verteilung der Impedanzwerte in das Röntgenbild zu projizieren. Weiter besteht die Möglichkeit, zweidimensionale Untermengen der dreidimensionalen Verteilung der Impedanzwerte, vorzugsweise parallel zu der Ebene des Röntgenbildes verlaufende Schichten der dreidimensionalen Impedanzwerte, in das Röntgenbild zu projizieren wobei die Koordinate z_{EI} der zu projizierenden Schicht mittels der Tastatur 14 gewählt werden kann.

Alternativ kann der Bildrechner 16 ein Fusionsbild dadurch erzeugen, daß er das Röntgenbild in eine vom Benutzer über die Tastatur 14 vorgebbare Ebene der dreidimensionalen Verteilung der Impedanzwerte, deren Koordinate z_{EI} mittels der Tastatur 14 gewählt werden kann, einblendet. Dabei rechnet der Bildrechner 16 zuvor die dem Röntgenbild entsprechenden digitalen Daten derart um, daß die erhaltenen Daten der Darstellung des Röntgenbildes in der der gewählten Schicht entsprechenden Ebene entsprechen.

Unabhängig davon, auf welche Weise ein Fusionsbild erzeugt wird, bietet es gegenüber herkömmlichen Röntgenbildern oder herkömmlichen, auf der Basis von elektrischen Impedanzmessungen gewonnenen Bildern erweiterte diagnostische Möglichkeiten, daß sofort erkennbar ist, ob Auffälligkeiten in dem Röntgenbild entsprechende Auffälligkeiten in der dreidimensionalen Verteilung der Impedanzwerte entsprechen.

Dabei sind die gewonnenen Fusionsbilder im Falle der erfindungsgemäßen Vorrichtung deshalb besonders aussagefähig, weil der zu untersuchende Gewebebereich während der Röntgenuntersuchung und während der elektrischen Impedanzmessung die gleiche Position in der Kompressionseinrichtung einnimmt. Dies gilt in besonders hohem Maße, wenn die Röntgenuntersuchung und die elektrische Impedanzmessung gleichzeitig durchgeführt werden.

Es besteht übrigens die Möglichkeit, zusätzlich zu der Kompressionsplatte 4 und dem Strahlenempfänger 3 weitere laterale, mit Elektrodenanordnungen versehene Kompressionsplatten vorzusehen, so daß die Rekonstruktion der dreidimensionalen Verteilung der Impedanzwerte nicht nur bezüglich einer ventral-dorsalen Achse (Brustwarze-Rippenauftreffpunkt) doppelseitig, sondern dann allseitig möglich ist.

Im Falle des dargestellten Ausführungsbeispiels dient die erfindungsgemäße Vorrichtung zur Untersuchung einer weiblichen Brust. Es können aber beliebige andere Gewebebereiche beliebiger Lebewesen mit der erfindungsgemäßen Vorrichtung untersucht werden.

## Patentansprüche

1. Vorrichtung zur Untersuchung von lebendem Gewebe durch Röntgenstrahlung und elektrische Impedanzmessung aufweisend:
- eine Röntgenstrahlenquelle (2),
- eine der Röntgenstrahlenquelle (2) gegenüberliegend angeordnete Strahlenempfangseinrichtung (3) für von der Röntgenstrahlungsquelle (2) ausgehende Röntgenstrahlung,
- eine Kompressionseinrichtung (3, 4) für das zu untersuchende Gewebe,
- eine an der Kompressionseinrichtung (3, 4) derart angebrachte Elektrodenanordnung (5, 6), daß Einzelelektroden (7₁ bis 7₁₂) der Elektrodenanordnung (5, 6) im Betrieb der Vorrichtung mit komprimiertem Gewebe in elektrischer Wechselwirkung stehen,
- Mittel zur Beaufschlagung (11) wenigstens eines Teils der Einzelelektroden (7₁ bis 7₁₂) mit Stromsignalen oder Spannungssignalen,
- Mittel zum Messen von durch die Stromsignale bzw. Spannungssignale in das im Betrieb der Vorrichtung komprimierte Gewebe induzierten Potentialen bzw. Strömen, welche wenigstens mit einem Teil der Einzelelektroden (7₁ bis 7₁₂) zusammenwirken, und
- eine Steuereinrichtung, welche die Röntgenstrahlenquelle (2) und die Strahlenempfangseinrichtung (3) einerseits und die Mittel zur Beaufschlagung (11) mit Stromsignalen oder Spannungssignalen und die Mittel zum Messen (12) von induzierten Potentialen bzw. Strömen andererseits wahlweise derart aktiviert, daß die Erzeugung eines Röntgenbildes und eine Messung von induzierten Potentialen bzw. Strömen entweder gleichzeitig oder sequentiell erfolgen.

2. Vorrichtung nach Anspruch 1, bei der die Einzelelektroden (7₁ bis 7₁₂) aus einem Röntgenstrahlung wenig schwächendem Material gebildet sind.

3. Vorrichtung nach Anspruch 1 oder 2, bei der die Elektrodenanordnung (5, 6) einen elektrisch nicht aktiven Bereich (9) aufweist, der derart geformt ist, daß er bei Abbildung mittels der von der Röntgenstrahlungsquelle (2) ausgehende Röntgenstrahlung auf der Strahlenempfangseinrichtung (3) ein dem Bild der Einzelelektroden (7₁ bis 7₁₂) inverses Bild ergibt, das wenigstens im wesentlichen den gleichen Schwärzungsgrad wie das Bild der Einzelelektroden (7₁ bis 7₁₂) aufweist.

4. Vorrichtung nach einem der Ansprüche 1 bis 3, bei der der im Nutzstrahlengang der von der Röntgenstrahlungsquelle (2) ausgehenden Röntgenstrahlung befindliche Bereich der Kompressionseinrichtung (3, 4) Röntgenstrahlung wenig schwächt.

5. Vorrichtung nach einem der Ansprüche 1 bis 4, bei der eine Recheneinrichtung (15) vorgesehen ist, die auf Grundlage von den mittels der Mittel zum Messen (12) von induzierten Potentialen bzw. Strömen gemessenen Potentialen bzw. Strömen entsprechenden digitalen Daten eine Verteilung von Impedanzwerten errechnet.

6. Vorrichtung nach Anspruch 5, bei der die Recheneinrichtung (15) eine dreidimensionale Verteilung von Impedanzwerten errechnet.

7. Vorrichtung nach Anspruch 5 oder 6, bei der die Recheneinrichtung (15) die Verteilung von Impedanzwerten durch ein Rekonstruktionsverfahren errechnet.

8. Vorrichtung nach einem der Ansprüche 5 bis 7, bei der die Recheneinrichtung (15) unter Berücksichtigung der der Erzeugung des Röntgenbildes und der Ermittlung der dreidimensionalen Verteilung der Impedanzwerte zugrundeliegenden Koordinatensysteme (x_{EI}, y_{EI}, z_{EI}, x_{R}, y_{R}) eine Fusion zumindest eines Teils der dreidimensionalen Verteilung der Impedanzwerte mit dem Röntgenbild entsprechenden digitalen Daten zu einem Fusionsbild bewirkt.

9. Vorrichtung nach einem der Ansprüche 5 bis 8, bei der die Recheneinrichtung (15) das Fusionsbild durch Projektion der dreidimensionalen Verteilung der Impedanzwerte in das Röntgenbild erzeugt.

10. Vorrichtung nach einem der Ansprüche 5 bis 9, bei der die Recheneinrichtung (15) das Fusionsbild durch Projektion einer oder mehrerer Schichten der dreidimensionalen Verteilung der Impedanzwerte in das Röntgenbild erzeugt.

11. Vorrichtung nach einem der Ansprüche 5 bis 10, bei der die Recheneinrichtung (15) das Fusionsbild durch Einblendung des Röntgenbildes in eine der Schichten der dreidimensionalen Verteilung der Impedanzwerte erzeugt.

12. Vorrichtung nach einem der Ansprüche 1 bis 11, bei der die Strahlenempfangseinrichtung (3) dem Röntgenbild entsprechende digitale Daten liefert, die einer Recheneinrichtung (15) zugeführt sind.

## Claims

1. Apparatus for examining living tissue by means of X-rays and electrical impedance measurement, comprising:
- an X-ray source (2),
- a radiation receiver (3), disposed opposite the X-ray source (2), for receiving the X-radiation emitted by the X-ray source (2),
- a compression device (3, 4) for compressing the tissue to be examined,
- an electrode arrangement (5, 6) disposed on the compression device (3, 4) such that individual electrodes (7₁ to 7₁₂) of the electrode arrangement (5, 6) interact electrically with compressed tissue during operation of the apparatus,
- means (11) of applying current signals or voltage signals to at least some of the individual electrodes (7₁ to 7₁₂),
- means of measuring potentials or currents induced in the compressed tissue by the current signals or voltage signals during operation of the apparatus, said potentials or currents interacting with at least some of the individual electrodes (7₁ to 7₁₂), and
- a control device which optionally activates the X-ray source (2) and the radiation receiver (3) on the one hand and the means (11) of applying current signals or voltage signals and the means (12) of measuring induced potentials or currents on the other, such that generation of an X-ray image and measurement of induced potentials or currents take place either simultaneously or sequentially.

2. Apparatus according to Claim 1, wherein the individual electrodes (7₁ to 7₁₂) are comprised of a material which is substantially transparent to X-rays.

3. Apparatus according to Claim 1 or 2, wherein the electrode arrangement (5, 6) has an electrically inactive region (9) formed such that during imaging it produces by means of the X-rays emitted by the X-ray source (2) an image on the radiation receiver (3) which is the inverse of that of the individual electrodes (7₁ to 7₁₂) and having essentially the same optical density as the image of the individual electrodes (7₁ to 7₁₂).

4. Apparatus according to one of Claims 1 to 3, wherein the region of the compression device (3, 4) lying in the path of the X-rays emitted by the X-ray source (2) is substantially transparent to X-rays.

5. Apparatus according to one of Claims 1 to 4, wherein a computing device (15) is provided which calculates a distribution of impedance values on the basis of digital data corresponding to the potentials or currents measured using the means (12) of measuring induced potentials or currents.

6. Apparatus according to Claim 5, wherein the computing device (15) calculates a three-dimensional distribution of the impedance values.

7. Apparatus according to Claim 5 or 6, wherein the computing device (15) calculates the distribution of impedance values by means of a reconstruction technique.

8. Apparatus according to one of Claims 5 to 7, wherein the computing device (15) fuses at least a portion of the three-dimensional distribution of impedance values with the digital data corresponding to the X-ray image to form a fusion image, taking into account the coordinate systems (x_{EI}, y_{EI}, z_{EI}, x_{R}, y_{R}) on which the generation of the X-ray image and the three-dimensional determination of the impedance values are based.

9. Apparatus according to one of Claims 5 to 8, wherein the computing device (15) produces the fusion image by projecting the three-dimensional distribution of impedance values into the X-ray image

10. Apparatus according to one of Claims 5 to 9, wherein the computing device (15) produces the fusion image by projecting one or more layers of the three-dimensional distribution of impedance values into the X-ray image.

11. Apparatus according to one of Claims 5 to 10, wherein the computing device (15) produces the fusion image by inserting the X-ray image into one of the layers of the three-dimensional distribution of impedance values.

12. Apparatus according to one of Claims 1 to 11, wherein the radiation receiver (3) supplies digital data corresponding to the X-ray image which is fed to a computing device (15).

## Revendications

1. Dispositif d'examen de tissu vivant par rayons X et de mesure d'impédance électrique comportant
- une source de rayons X (2),
- un dispositif de réception de rayons (3) disposé en face de la source de rayons X (2) et destiné à recevoir les rayons X émis par la source de rayons X (2),
- un dispositif de compression (3, 4) du tissu à examiner,
- un agencement d'électrodes (5, 6) monté sur le dispositif de compression (3, 4) de sorte que des électrodes individuelles (7₁ à 7₁₂) de l'agencement d'électrodes (5, 6) soient, lorsque le dispositif est en fonctionnement, en interaction électrique avec du tissu comprimé,
- des moyens (11) pour exciter au moins une partie des électrodes individuelles (7₁ à 7₁₂) par des signaux de courant ou des signaux de tension,
- des moyens pour mesurer des potentiels ou des courants induits par les signaux de tension ou par les signaux de courant dans le tissu comprimé, lorsque le dispositif est en fontionnement, lesquels potentiels ou courants coopèrent au moins avec une partie des électrodes individuelles (7₁ à 7₁₂), et
- un dispositif de commande, qui active d'une part la source de rayons X (2) et le dispositif de réception de rayons (3) et d'autre part les moyens (11) pour exciter par des signaux de courant ou des signaux de tension et les moyens (12) pour mesurer au choix des potentiels ou des courants induits de sorte que la génération d'une image formée par rayons X et une mesure de potentiels ou de courants induits sont effectuées simultanément ou séquentiellement.

2. Dispositif selon la revendication 1, dans lequel les électrodes individuelles (7₁ à 7₁₂) sont en un matériau atténuant peu les rayons X.

3. Dispositif selon la revendication 1 ou 2, dans lequel l'agencement d'électrodes (5, 6) comporte une zone (9) électriquement inactive qui est formée de manière à délivrer une image inverse de l'image des électrodes individuelles (7₁ à 7₁₂) lors de la reproduction au moyen des rayons X émis par la source de rayons X (2) sur le dispositif de réception de rayons (3), laquelle image a une intensité de noircissement au moins sensiblement identique à celle de l'image des électrodes individuelles (7₁ à 7₁₂).

4. Dispositif selon l'une des revendications 1 à 3, dans lequel la zone du dispositif de compression (3, 4) qui se trouve sur la trajectoire du faisceau actif des rayons X émis par la source de rayons X (2) atténue peu les rayons X.

5. Dispositif selon l'une des revendications 1 à 4, dans lequel il est prévu un dispositif de calcul (15) qui calcule une distribution des valeurs d'impédance à partir de données numériques correspondant à des potentiels ou des courants mesurés par les moyens (12) de mesure de potentiels ou de courants induits.

6. Dispositif selon la revendication 5, dans lequel le dispositif de calcul (15) calcule une distribution tridimensionnelle de valeurs d'impédance.

7. Dispositif selon la revendication 5 ou 6, dans lequel le dispositif de calcul (15) calcule la distribution de valeurs d'impédance par un procédé de reconstruction.

8. Dispositif selon l'une des revendications 5 à 7, dans lequel le dispositif de calcul (15) réalise une fusion d'au moins une partie de la distribution tridimensionnelle des valeurs d'impédance avec des données numériques correspondant à l'image formée par rayons X en vue d'obtenir une image de fusion en tenant compte des systèmes de coordonnées (x_{EI}, y_{EI}, z_{EI}, x_{R}, y_{R}) à la base de la génération de l'image formée par rayons X et de la détermination de la distribution tridimensionnelle des valeurs d'impédances.

9. Dispositif selon l'une des revendications 5 à 8, dans lequel le dispositif de calcul (15) génère l'image de fusion par projection de la distribution tridimensionnelle des valeurs d'impédance dans l'image formée par rayons X.

10. Dispositif selon l'une des revendications 5 à 9, dans lequel le dispositif de calcul (15) génère l'image de fusion par projection d'une ou plusieurs couches de la distribution tridimensionnelle des valeurs d'impédance dans l'image formée par rayons X.

11. Dispositif selon l'une des revendications 5 à 10, dans lequel le dispositif de calcul (15) génère l'image de fusion en insérant l'image formée par rayons X dans une des couches de la distnbution tridimensionnelle des valeurs d'impédance.

12. Dispositif selon l'une des revendications 1 à 11, dans lequel le dispositif de réception de rayons (3) délivre des données numériques qui correspondent à l'image formée par rayons X et qui sont acheminées à un dispositif de calcul (15).
